# DEMANDE DE BREVET EUROPEEN

(11) **EP 1 129 697 A1**
(43) Date de publication de la demande: **05.09.2001**
(21) Numéro de dépôt: 00403278.5
(22) Date de dépôt: 23.11.2000
(51) Int. Cl.: A61K 7/48, A61K 47/14

(54) **Composition, notamment cosmétique, comprenant un acide triterpène pentacyclique**

(30) Priorité: 10.12.1999 FR 9915612
(71) Demandeur: L'OREAL, 75008 Paris (FR)
(72) Inventeur: Leroy, Laurence, 78120 Rambouillet (FR); Burnier, Véronique, 75011 Paris (FR)
(74) Mandataire: Renard, Emmanuelle

(57) **Abrégé**

L'invention concerne une composition pour application topique comprenant, dans un milieu physiologiquement acceptable, au moins 0,02% d'au moins un acide triterpène pentacyclique et au moins un émulsionnant liquide à 25°C, qui est monoester d'acide gras éventuellement oxyalkyléné, ayant un HLB supérieur ou égal à 12.

L'acide triterpène pentacyclique est de préférence choisi dans le groupe constitué de l'acide ursolique et de l'acide oléanolique.

## Description

La présente invention se rapporte à un procédé de solubilisation d'un acide triterpène pentacyclique. Elle se rapporte également à de nouvelles compositions comprenant, dans un milieu physiologiquement acceptable, au moins un acide triterpène pentacyclique et au moins un émulsionnant liquide à 25°C, qui est monoester d'acide gras ayant un HLB supérieur ou égal à 12. Elle se rapporte aussi à l'utilisation des compositions précitées dans une préparation cosmétique, ou pour la fabrication d'une préparation destinée en particulier au soin de la peau, des muqueuses et/ou des fibres kératiniques.

Les acides triterpène pentacycliques tels que l'acide ursolique et l'acide oléanolique sont présents dans les plantes telles que le romarin. Ils sont fréquemment utilisés dans les compositions pharmaceutiques pour leurs nombreuses propriétés thérapeutiques, et par exemple pour leurs propriétés anti-inflammatoires, hépatoprotectrices, diurétiques, analgésiques, antimicrobiennes, inhibitrices d'activité enzymatique et anti-tumorales. Ils sont ainsi décrits dans le document JP-2017121 pour la prévention du cancer de la peau.
Dans le domaine cosmétique, l'acide ursolique est décrit par exemple comme constituant d'une composition anti-transpirante dans le document FR-A-2 541 895 et comme inhibiteur de l'activité de la tyrosinase dans le document JP-58/57307.

Les acides triterpène pentacycliques sont donc d'un grand intérêt, étant donnés leurs effets biologiques sur la peau. Toutefois, la formulation de ces composés est problématique dans la mesure où ces composés se présentent sous forme de cristaux insolubles ou difficilement solubles dans l'eau et dans les huiles ou solvants traditionnellement utilisés dans les domaines cosmétique et dermatologique. Cette faible solubilité des acides triterpène pentacycliques nuit à l'efficacité des compositions les contenant et à leur perception par les utilisateurs.

Ainsi, selon le document de Jin et al., Arch. Pharm. Res., 1997, Vol. 20, n°3, p. 275 à 279, les tensioactifs et la plupart des solvants (tels que les polyéthylène glycols) sont peu appropriés ou permettent seulement une solubilisation limitée de l'acide ursolique.

Il a cependant été proposé de solubiliser les acides triterpène pentacycliques dans des solutions aqueuses concentrées en tensioactifs ioniques ou en tensioactifs non ioniques constitués d'éthers d'alcools gras et de polyéthylène glycol. L'emploi de telles quantités de ce type de tensioactifs n'est cependant pas approprié à la réalisation de compositions cosmétiques puisqu'elles provoquent des phénomènes d'irritation, d'échauffement et de rougeurs inacceptables pour une composition cosmétique qui doit être exempte de tout effet secondaire susceptible de détourner l'utilisateur de son emploi.

Un autre solvant proposé dans la publication ci-dessus pour solubiliser les acides triterpène pentacycliques est la tétraméthyl urée. Outre que son utilisation est limitée en cosmétique pour des problèmes de tolérance cutanée, la tétraméthyl urée ne constitue un bon solubilisant de l'acide ursolique que dans le cas où la quantité d'eau présente dans la composition est inférieure à 30%, voire à 20%. Or, les compositions cosmétiques sous forme de fluides ou de lotion non gras, voire de crèmes, renferment fréquemment plus de 50% en poids d'eau. Pour cette double raison, la tétraméthyl urée ne convient pas à un usage cosmétique.

Le document JP-2 017 121 divulgue des exemples de compositions cosmétiques renfermant de l'acide ursolique et/ou de l'acide oléanolique. Dans l'Exemple 1, une crème est préparée par mélange d'une solution hydroalcoolique d'acide ursolique neutralisé, chauffée à 80°C, avec un mélange de corps gras fondus porté à la même température. L'acide ursolique représente 0,01% en poids de la composition totale. Le mélange de corps gras contient un mono-stéarate de sorbitane polyoxyéthyléné, qui est vraisemblablement soit solide (du type Tween 61®), soit semi-solide (du type Tween 60®). Ce type d'émulsionnant ne permet pas de solubiliser une quantité suffisante d'acide ursolique sans perte de stabilité de la composition, en particulier lorsque l'acide ursolique se trouve sous forme d'acide libre.

Il subsiste donc le besoin de pouvoir introduire une quantité d'au moins 0,02% en poids d'acide triterpène pentacyclique dans une composition, notamment cosmétique, qui soit stable et sensiblement non-irritante, dans laquelle l'acide triterpénique n'aura pas tendance à recristalliser pendant la durée de conservation et d'utilisation de la composition.

La Demanderesse a découvert, de façon inattendue, que ce besoin était satisfait, selon l'invention, par l'utilisation de certains monoesters d'acides gras.

La présente invention a donc pour objet une composition pour application topique comprenant, dans un milieu physiologiquement acceptable, au moins 0,02% d'au moins un acide triterpène pentacyclique et au moins un émulsionnant liquide à 25°C, qui est monoester d'acide gras éventuellement oxyalkyléné, ayant un HLB supérieur ou égal à 12.

L'acide triterpène pentacylique est de préférence choisi parmi l'acide ursolique et l'acide oléanolique. Il se trouve de préférence essentiellement sous forme d'acide libre, c'est-à-dire à hauteur d'au moins 50% en poids.

On entend de préférence par monoester d'acide gras, un monoester formé à partir d'un acide gras ayant de 12 à 22 atomes de carbone et soit d'un polyol, éventuellement oxyalkyléné, soit d'un polyalkylèneglycol, l'ester obtenu étant suffisamment hydrophile pour que son HLB soit supérieur ou égal à 12, de préférence compris entre 12 et 17. On préfère en particulier les monoesters d'acide gras formés à partir d'un acide gras comprenant de 12 à 22 atomes de carbone et de polyglycérol, et les monoesters d'acide gras formés à partir d'un acide gras comprenant de 12 à 22 atomes de carbone et de sorbitane polyoxyéthyléné.

L'invention a encore pour objet un procédé de solubilisation d'un acide triterpène pentacylique, comprenant l'étape consistant à mélanger à température ambiante ledit acide avec au moins un monoester d'acide gras éventuellement oxyalkyléné, liquide à 25°C et ayant un HLB supérieur ou égal à 12.

Le monoester d'acide gras est présent, dans la composition selon l'invention, en une quantité suffisante pour permettre la solubilisation de l'acide triterpène pentacyclique et obtenir une composition stable, sans recristallisation du triterpène. Par "stable", on entend de préférence selon l'invention que l'acide triterpène pentacyclique ne recristallise pas lorsque la composition est maintenue au moins quatre semaines à température ambiante. On entend également l'absence de déphasage, crémage, sédimentation ou autre phénomène traduisant une instabilité de la composition elle-même.

Bien entendu, la quantité de monoester d'acide gras dépendra de la quantité d'acide triterpène pentacylique. A titre indicatif, l'acide triterpène pentacylique représente de préférence de 0,05 à 5%, mieux, de 0,4 à 2%, encore mieux, environ 1 %, du poids total de la composition. Dans ces conditions, le monoester d'acide gras représente avantageusement de 0,05 à 10% et, de préférence, de 1 à 5%, du poids total de la composition.

Les acides triterpène pentacycliques utilisés selon l'invention sont de préférence constitués de l'acide ursolique et/ou de l'acide oléanolique.

Comme monoesters d'acide gras, on peut citer:
- les monoesters d'acide gras et de polyglycérol, tels que le mono-laurate d'hexaglycéryle, le mono-isostéarate de décaglycéryle, le mono-laurate de décaglycéryle, le monolinoléate de décaglycéryle, le mono-myristate de décaglycéryle et le mono-oléate de décaglycéryle ;
- les monoesters d'acide gras et de sorbitane/sorbitol polyoxyéthylénés, tels que le mono-stéarate de sorbitane polyoxyéthyléné, le mono-laurate de sorbitane polyoxyéthyléné (20 OE), le monolaurate de sorbitane polyoxyéthyléné (4 OE), le mono-oléate de sorbitane polyoxyéthyléné (20 OE), le tétra-oléate de sorbitol polyoxyéthyléné (40 OE) et le tétra-oléate de sorbitol polyoxyéthyléné (60 OE) ; et
- les monoesters d'acide gras et de polyalkylène glycol, tels que le mono-oléate de polyéthylène glycol (600 OE), le mono-laurate de polyéthylène glycol (400 OE), l'huile de ricin polyoxyéthylénée (40 OE), l'huile de ricin polyoxyéthylénée (60 OE) et l'huile de ricin hydrogénée polyoxyéthylénée (40 OE).

Ces composés ont l'avantage d'éviter la recristallisation des acides triterpène pentacycliques et de permettre leur incorporation dans la phase aqueuse de compositions cosmétiques ou dermatologiques, sans entraîner d'irritation cutanée.

A cet égard, il a été découvert que l'acide triterpène pentacyclique pouvait être incorporé, sans recristallisation, dans des compositions contenant de grandes quantités de phase aqueuse. Ainsi, la composition selon l'invention peut par exemple contenir de 30 à 95% en poids d'eau, avantageusement de 50 à 80% en poids d'eau.

Cette phase aqueuse peut contenir en plus notamment un ou plusieurs alcools et/ou polyols tels que l'éthanol, la glycérine, le butylène glycol, l'isoprène glycol, le propylène glycol, le sorbitol, dans des concentrations allant préférentiellement de 1 à 20% en poids, par rapport au poids total de la composition.

La composition selon l'invention peut se présenter sous toute forme galénique appropriée à une utilisation topique et notamment sous forme d'une solution aqueuse, d'un gel aqueux, d'une dispersion, d'une émulsion huile-dans-eau (H/E) ou eau-dans-huile (E/H), d'une émulsion triple (E/H/E ou H/E/H). Elles peuvent également se trouver sous une forme vectorisée, comme par exemple sous forme de nanocapsules, de liposomes, de nanoémulsions ou d'oléosomes.

Selon un mode préféré de réalisation de l'invention, la composition est une émulsion E/H ou H/E.

Cette composition est destinée à une application topique et comprend de manière appropriée un milieu physiologiquement acceptable. Par milieu physiologiquement acceptable, on entend un milieu compatible avec la peau, les muqueuses (dont les lèvres) et les phanères (ongles, cheveux et cils).

Lorsque la composition est une émulsion, cette dernière contient de façon classique au moins une huile, une phase aqueuse et éventuellement un émulsionnant adéquat, en plus du monoester d'acide gras selon l'invention.

La nature de la phase huileuse entrant dans la composition selon l'invention n'est pas critique et elle peut ainsi être constituée par tous les corps gras et notamment les huiles, classiquement utilisés dans les domaines cosmétique et dermatologique.

Parmi les huiles utilisables dans l'émulsion de l'invention, on peut notamment citer les huiles végétales, les huiles minérales, les huiles de synthèse, les huiles de silicone et les huiles fluorées. Les autres corps gras susceptibles d'être présents dans la phase huileuse peuvent être par exemple les acides gras, les alcools gras et les cires.

La phase huileuse de l'émulsion peut représenter de 1 à 70% et, mieux, de 5 à 40% du poids total de l'émulsion.

Les émulsions peuvent contenir au moins un agent émulsionnant, autre que le monoester d'acide gras selon l'invention, permettant de stabiliser l'interface huile/eau. Cet émulsionnant supplémentaire peut être choisi parmi les agents émulsionnants anioniques, cationiques ou non ioniques, utilisés seuls ou en mélange. Ces émulsionnants supplémentaires sont choisis de façon appropriée en fonction de l'émulsion à obtenir (E/H ou H/E).

Selon la fluidité que l'on souhaite conférer à la composition, on peut également y ajouter un ou plusieurs gélifiants qui peuvent représenter, par exemple, de 0,1 à 10%, et de préférence de 0,1 à 5%, mieux, de 0,1 à 3% du poids total de la composition.

En outre, de façon connue, la composition de l'invention peut contenir des adjuvants habituels dans les domaines cosmétique et dermatologique, tels que les actifs hydrophiles ou lipophiles, les conservateurs, les antioxydants, les parfums, les charges, les matières colorantes (pigments ou colorants) et les filtres solaires. Ces adjuvants sont utilisés dans les proportions habituelles dans les domaines cosmétique et dermatologique, et par exemple à raison de 0,01 à 20% du poids total de la composition. Selon leur nature, ils sont introduits dans la phase aqueuse ou dans la phase huileuse de la composition, ou encore dans des vésicules. La nature et la quantité de ces adjuvants doivent cependant être telles qu'elles ne modifient pas les propriétés recherchées pour la composition selon l'invention.

La composition selon l'invention est avantageusement obtenue selon un procédé comprenant l'étape consistant à mélanger à température ambiante, généralement en présence d'eau, l'acide triterpène pentacylique avec le monoester d'acide gras éventuellement oxyalkyléné, liquide à 25°C et ayant un HLB supérieur ou égal à 12.

Ce procédé comprend de préférence les deux étapes supplémentaires consistant à homogénéiser le mélange à température ambiante, puis à l'introduire dans une émulsion H/E ou E/H, également à température ambiante.

La composition selon l'invention trouve une application dans un grand nombre de traitements cosmétiques et dermatologiques de la peau, des muqueuses et/ou des phanères, notamment pour la protection, le soin, le nettoyage et/ou le maquillage de la peau et/ou des muqueuses, pour la protection et/ou le soin des cheveux et/ou pour le traitement dermatologique de la peau, des cheveux et/ou des muqueuses.

A cet effet, la composition selon l'invention peut être utilisée dans des produits de soin ou de nettoyage pour le visage ou le corps, sous forme de lotions, de crèmes ou de laits, dans des produits de maquillage par incorporation de charges, de pigments ou de colorants, ou encore dans des produits solaires, par incorporation de filtres solaires.

Ainsi, l'invention a pour objet un procédé cosmétique de prévention ou de traitement de l'acné, du photovieillissement et/ou des peaux sensibles, comprenant l'application topique de la composition décrite précédemment.

L'invention a également pour objet l'utilisation de la composition décrite précédemment pour la fabrication d'une préparation destinée à la prévention ou au traitement de l'acné et/ou de l'érythème solaire et/ou des peaux sensibles.

D'autres caractéristiques et avantages de l'invention ressortiront mieux des exemples de réalisation qui suivent, donnés à titre illustratif et non limitatif. Dans ces exemples, sauf indication contraire, les quantités sont indiquées en pourcentage pondéral.

### EXEMPLES

### Exemple 1 : Emulsion H/E

| *Phase A* | |
|---|---|
| Stéarate de polyoxyéthylèneglycol (100 OE) et stéarate de glycéryle | 2,5% |
| Mono-stéarate de polyéthylèneglycol (50 OE) | 2,5% |
| Alcool stéarylique | 1 % |
| Alcool cétylique | 1 % |
| Polyisobutène hydrogéné | 20 % |

| *Phase B* | |
|---|---|
| Carbomer (polymère carboxyvinylique) | 0,3% |
| Triéthanolamine | 0,3% |
| Eau | 10 % |

| *Phase C* | |
|---|---|
| Mono-oléate de sorbitane | 4 % |
| Acide ursolique (fourni par NIKKOL) | 1,6% |
| Eau | qsp 34,4% |

| *Phase D* | |
|---|---|
| Conservateurs | qs |
| Eau | qsp. 100 % |

La composition ci-dessus a été préparée de la manière suivante. On a d'abord préparé une émulsion huile-dans-eau, de manière classique, en introduisant, sous agitation, à 70-75°C, la phase A dans la phase D. La phase B a été préparée à 70-75°C, puis ajoutée à 40-50°C, sous agitation, à l'émulsion précédemment obtenue. La phase C a été préparée par mélange de ses constituants à température ambiante et homogénéisation pendant 48 heures, puis elle a été ajoutée, sous agitation, au mélange des phases A, B et D précédemment refroidi à température ambiante.

On n'observe pas de recristallisation de l'acide ursolique après un mois à température ambiante et à 45°C.

### Exemple 2 : Emulsion H/E

On a préparé la composition suivante de la même manière que dans l'Exemple 1.

| *Phase A* | |
|---|---|
| Stéarate de polyoxyéthylèneglycol (100 OE) et stéarate de glycéryle | 2,5% |
| Mono-stéarate de polyéthylèneglycol (50 OE) | 2,5% |
| Alcool stéarylique | 1 % |
| Alcool cétylique | 1 % |
| Polyisobutène hydrogéné | 20 % |

| *Phase B* | |
|---|---|
| Carbomer (polymère carboxyvinylique) | 0,3% |
| Triéthanolamine | 0,3% |
| Eau | 10 % |

| *Phase C* | |
|---|---|
| Mono-oléate polyglycérolé | 1 % |
| Acide ursolique (fourni par NIKKOL) | 0,4% |
| Eau | qsp 8,6% |

| *Phase D* | |
|---|---|
| Conservateurs | qs |
| Eau | qsp. 100 % |

On n'observe pas de recristalisation de l'acide ursolique après un mois à température ambiante et à 45°C.

### Exemple 3 (Comparatif)

On a préparé la composition suivante de la même manière que dans l'Exemple 1.

| *Phase A* | |
|---|---|
| Stéarate de polyoxyéthylèneglycol (100 OE) et stéarate de glycéryle | 2,5% |
| Mono-stéarate de polyéthylèneglycol (50 OE) | 2,5% |
| Alcool stéarylique | 1 % |
| Alcool cétylique | 1 % |
| Polyisobutène hydrogéné | 20 % |

| *Phase B* | |
|---|---|
| Carbomer (polymère carboxyvinylique) | 0,3% |
| Triéthanolamine | 0,3% |
| Eau | 10 % |

| *Phase C* | |
|---|---|
| Di-oléate polylgycérolé | 1 % |
| Acide ursolique (fourni par NIKKOL) | 0,4% |
| Eau | qsp 8,6% |

| *Phase D* | |
|---|---|
| Conservateurs | qs |
| Eau | qsp. 100 % |

On observe l'apparition d'amas cristallins après un mois à température ambiante et à 45°C.

## Revendications

1. Composition pour application topique comprenant, dans un milieu physiologiquement acceptable, au moins 0,02% d'au moins un acide triterpène pentacyclique et au moins un émulsionnant liquide à 25°C, qui est monoester d'acide gras éventuellement oxyalkyléné, ayant un HLB supérieur ou égal à 12.

2. Composition selon la revendication 1, caractérisée en ce que ledit acide triterpène pentacyclique est choisi parmi l'acide ursolique et l'acide oléanolique.

3. Composition selon la revendication 1 ou 2, caractérisée en ce que ledit acide triterpène pentacyclique se trouve essentiellement sous forme d'acide libre.

4. Composition selon l'une quelconque des revendications 1 à 3, caractérisée en ce que ledit monoester d'acide gras est formé à partir d'un acide gras comprenant de 12 à 22 atomes de carbone et d'un polyol éventuellement oxyéthyléné.

5. Composition selon la revendication 4, caractérisée en ce que ledit monoester d'acide gras est formé à partir d'un acide gras comprenant de 12 à 22 atomes de carbone et de polyglycérol.

6. Composition selon la revendication 4, caractérisée en ce que ledit monoester d'acide gras est formé à partir d'un acide gras comprenant de 12 à 22 atomes de carbone et de sorbitane polyoxyéthyléné.

7. Composition selon l'une quelconque des revendications 1 à 6, caractérisée en ce que l'acide triterpène pentacylique représente de 0,05 à 5% du poids total de la composition.

8. Composition selon l'une quelconque des revendications 1 à 7, caractérisée en ce que l'acide triterpène pentacylique représente de 0,4 à 2% du poids total de la composition.

9. Composition selon l'une quelconque des revendications 1 à 8, caractérisée en ce que le monoester d'acide gras représente de 0,05 à 10% du poids total de la composition.

10. Composition selon l'une quelconque des revendications 1 à 9, caractérisée en ce que le monoester d'acide gras représente de 1 à 5% du poids total de la composition.

11. Composition selon l'une quelconque des revendications 1 à 10, caractérisée en ce qu'elle contient de 30 à 95% en poids d'eau.

12. Composition selon la revendication 11, caractérisée en ce qu'elle renferme de 50 à 80% en poids d'eau.

13. Procédé de solubilisation d'un acide triterpène pentacylique, comprenant l'étape consistant à mélanger à température ambiante ledit acide avec au moins un monoester d'acide gras éventuellement oxyalkyléné, liquide à 25°C et ayant un HLB supérieur ou égal à 12.

14. Procédé selon la revendication 13, comprenant l'étape supplémentaire d'homogénéisation du mélange à température ambiante.

15. Procédé selon la revendication 14, comprenant l'étape supplémentaire d'introduction du mélange dans une émulsion H/E ou E/H, à température ambiante.

16. Procédé cosmétique de prévention ou de traitement de l'acné, du photovieillissement et/ou des peaux sensibles, comprenant l'application topique de la composition selon l'une quelconque des revendications 1 à 12.

17. Utilisation de la composition selon l'une quelconque des revendications 1 à 12 pour la fabrication d'une préparation destinée à la prévention ou au traitement de l'acné et/ou de l'érythème solaire et/ou des peaux sensibles.
